# EUROPÄISCHE PATENTANMELDUNG

(11) **EP 4 763 173 A1**
(43) Veröffentlichungstag der Anmeldung: **24.06.2026**
(21) Anmeldenummer: 25222735.0
(22) Anmeldetag: 11.12.2025
(51) Int. Cl.: A61G 7/002, A61G 7/018, A61B 5/103

(54) **BETTGESTELL FÜR EIN PFLEGEBETT, PFLEGEBETT UND EIN VERFAHREN ZUM BETREIBEN EINES PFLEGEBETTES UND EIN VERFAHREN ZUM KALIBRIEREN EINES PFLEGEBETTES**

(30) Priorität: 20.12.2024 DE 102024139349
(71) Anmelder: Nieuwenhuis, Günter, 53919 Weilerswist (DE)
(72) Erfinder: Nieuwenhuis, Günter, 53919 Weilerswist (DE)
(74) Vertreter: Patentanwälte Bauer Vorberg Kayser

(57) **Zusammenfassung**

Die Erfindung betrifft ein Bettgestell (1) für ein Pflegebett (2), umfassend einen Träger (10) und eine Mehrzahl von Latten (20), die jeweils zwei Endabschnitte (21, 22) aufweisen und zur Ausbildung einer Schlafoberflächenbasis (100) eingerichtet sind, wobei die Endabschnitte (21, 22) jeweils mittels einer jeweiligen motorischen Verstelleinrichtung (30) gegenüber dem Träger (10) in ihrer Vertikalposition verstellbar sind. Erfindungsgemäß weist jeder der Verstelleinrichtungen (30) eine Hebeleinrichtung (40) mit einem von der Vertikalposition abhängigen Übersetzungsverhältnis und eine Vorspanneinrichtung (50) auf, wobei die Vorspanneinrichtung (50) die Hebeleinrichtung (40) in eine Verstellrichtung (V) vorspannt. Weiterhin betrifft die Erfindung ein Pflegebett (2) und ein Verfahren zum Betreiben und ein Verfahren zum Kalibrieren eines Pflegebetts.

## Beschreibung

Die Erfindung betrifft ein Bettgestell für ein Pflegebett, umfassend einen Träger und eine Mehrzahl von Latten, die jeweils zwei Endabschnitte aufweisen und zur Ausbildung einer Schlafoberflächenbasis eingerichtet sind, wobei die Endabschnitte jeweils mittels einer jeweiligen motorischen Verstelleinrichtung gegenüber dem Träger in ihrer Vertikalposition verstellbar sind. Weiterhin betrifft die Erfindung ein Pflegebett, sowie ein Verfahren zum Betreiben eines Pflegebetts und ein Verfahren zum Kalibrieren eines Pflegebetts.

Aus der US 10 588 420 B1 ist ein Bettgestell für ein Pflegebett vorbekannt, bei dem eine Mehrzahl von Latten vorgesehen sind, die mittels einer als Spindeltrieb ausgebildeten Verstelleinrichtung in ihrer Vertikalposition verstellbar sind. Die Spindeltriebe weisen dazu eine Gewindespindel auf, die in Vertikalrichtung angeordnet ist. Nachteilig an dieser Anordnung ist es, dass diese einen hohen Bauraumbedarf, insbesondere in Vertikalrichtung, aufweist und dass Bettgestell somit nur unzureichend tief eingestellt werden kann, so dass beispielsweise ein Wechsel zwischen dem Pflegebett und einem Rollstuhl erschwert ist. Weiterhin ist es erforderlich, dass die Verstelleinrichtung ausreichend hohe Antriebskräfte bereitstellen kann und ausreichend stabil ausgeführt ist. Um ein Ausknicken der Gewindespindeln zu verhindern, müssen diese einen relativ großen Durchmesser aufweisen, ohne dass dies die eigentliche Getriebefunktion des Spindeltriebs erfordern würde. Weiterhin geht aus diesem Stand der Technik ein Verfahren zum Betreiben eines solchen Bettgestells hervor.

Weiterhin ist aus der DE 10 2019 007 458 A1 ein Antidekubitiusmodul für ein Pflegebett vorbekannt, das mit einer modularen Pflegebettmechanik mit einem Antrieb mit einer Schnittstelle und einen Stempel ausgestattet ist, wobei der Antrieb über die Schnittstelle mit einer Steuer- oder Regelungseinheit derart regel- bzw. ansteuerbar ist, dass der Stempel in eine definierbare Position/Stellung verschiebbar ist. Derartige Stempelanordnungen benötigen einen hohen Bauraumbedarf. Dies ist insbesondere dann der Fall, wenn hohe Verstellwege realisiert werden sollen, da die minimale Länge durch die ineinander teleskopierbaren Elemente der Stempelanordnung vorbestimmt ist.

Ausgehend von den zuvor beschriebenen Nachteilen des Standes der Technik ist es eine Aufgabe der vorliegenden Erfindung, ein Bettgestell für ein Pflegebett bzw. ein Pflegebett anzugeben, welches einen kompakten und stabilen Aufbau aufweist und welches gleichzeitig eine große, individuelle Verstellmöglichkeit jeder einzelnen Latte der Schlafoberflächenbasis bildet. Weiterhin ist es eine Aufgabe ein Verfahren zum Betreiben bzw. Kalibrieren eines Pflegebettes mit einem Bettgestell anzugeben, welches einen kompakten und gleichzeitig stabilen Aufbau aufweist.

Gelöst wird die Aufgabe durch die Merkmale der unabhängigen Patentansprüche.

Die Unteransprüche betreffen jeweils bevorzugte Ausgestaltungen bzw. Weiterbildungen der vorliegenden Erfindung, deren jeweilige Merkmale im Rahmen des technisch Sinnvollen ggf. auch über die Kategoriegrenzen der verschiedenen Ansprüche hinweg frei miteinander kombinierbar sind.

Es wird zur Lösung der Aufgabe ein Bettgestell für ein Pflegebett vorgeschlagen, umfassend einen Träger und eine Mehrzahl von Latten, die jeweils zwei Endabschnitte aufweisen und zur Ausbildung einer Schlafoberflächenbasis eingerichtet sind, wobei die Endabschnitte jeweils mittels einer jeweiligen motorischen Verstelleinrichtung gegenüber dem Träger in ihrer Vertikalposition verstellbar sind. Erfindungsgemäß weist jeder der Verstelleinrichtungen eine Hebeleinrichtung mit einem von der Vertikalposition abhängigen Übersetzungsverhältnis und eine Vorspanneinrichtung auf, wobei die Vorspanneinrichtung die Hebeleinrichtung in eine Verstellrichtung vorspannt.

Dank der erfindungsgemäßen Lösung kann ein Bettgestell für ein Pflegebett bereitgestellt werden, welches einen kompakten und gleichzeitig stabilen Aufbau aufweist.

Insbesondere ermöglicht die Kombination der Hebeleinrichtung mit der Vorspanneinrichtung, welche die Hebeleinrichtung in die Verstellrichtung vorspannt, eine kompakte und zugleich sehr stabile Bauweise.

Aufgrund der Wirkung der Vorspanneinrichtung, welche die in ihr gespeicherte Energie in die Hebeleinrichtung, auch als Hebelmechanismus bezeichnet, überträgt, wenn die Verstelleinrichtung eine Verstellung in die Verstellrichtung vornimmt, wird die von der motorischen Verstelleinrichtung zusätzlich zu verrichtende Verstellarbeit im Vergleich zu einem System ohne Vorspanneinrichtung reduziert. Die Vorspanneinrichtung dient dabei als Energiespeicher, vorzugsweise in Form eines Federspeichers, welche vorgespannt wird, wenn die Verstelleinrichtung den Endabschnitt der Latte in einem ersten Richtungssinn der Verstellrichtung verstellt. Bei einer Umkehr der Bewegungsrichtung, also bei einer Bewegung in den entgegengesetzten Richtungssinn, wird die in der Vorspanneinrichtung gespeicherte Energie in die Verstelleinrichtung zurückgeführt. Insbesondere wird die gespeicherte Energie in die Hebeleinrichtung zurückgeführt. Dadurch kann die motorische Verstelleinrichtung leistungsmäßig deutlich kleiner dimensioniert werden, wodurch sich Kostenvorteile ergeben. Darüber hinaus kann die motorische Verstelleinrichtung deutlich kompakter ausgeführt werden, auch insbesondere durch den Einsatz der Hebeleinrichtung.

Der Träger ist ein Strukturelement, wobei dieser bevorzugt als Rahmen ausgebildet ist, der die tragende Basis des Bettgestells bildet. Der bevorzugt als Rahmen ausgebildete Träger umfasst dabei bevorzugt eine Grundstruktur, die die Verstelleinrichtung und die Mehrzahl von Latten stützt und trägt.

In einer bevorzugten Ausführungsform ist der Träger als geschweißte oder verschraubte Rahmenkonstruktion aus Metall, insbesondere Stahl oder Aluminium, ausgeführt, wodurch eine hohe Belastbarkeit und Langlebigkeit gewährleistet werden. Alternativ kann der Träger auch aus einem hochfesten Kunststoff bestehen, um die Masse des Bettgestells zu reduzieren. Der Rahmen weist bevorzugt eine rechteckige oder quadratische Grundform auf, wobei die Abmessungen an die typischen Maße eines Bettgestells eines Pflegebetts angepasst sind.

Zusätzlich können derartige Träger mit Verstärkungsstreben oder Querverbindungen ausgestattet sein, um die Stabilität weiter zu erhöhen. Die Querschnitte der Profile des Trägers sind dabei vorzugsweise als Hohlprofile, U-Profile oder I-Profile ausgebildet, um eine optimale Kombination aus Masse und Steifigkeit zu erreichen.

Die Latte ist bevorzugt als längliches, biegeelastische Elemente ausgebildet, wobei die Mehrzahl der Latten parallel zueinander angeordnet und mittels der Verstelleinrichtung individuell einstellbar sind, wobei die Latten die stützende Basis für eine Schlafoberfläche (Schlafoberflächenbasis) bilden, auf der bevorzugt eine Matratze angeordnet werden kann. Jede der Latten weist zwei Endabschnitte auf, sprich einen ersten Endabschnitt und einen zweiten Endabschnitt.

Bevorzugt sind die Latten in definierten Abständen zueinander angeordnet, um eine ausreichende Belüftung der Schlafoberflächenbasis zu gewährleisten. Die Latten tragen somit zur ergonomischen Unterstützung und zur Feuchtigkeitsregulierung bei, wodurch ein höherer Komfort und eine bessere Hygiene sichergestellt ist.

In einer bevorzugten Ausführungsform umfassen die Latten ein flexibles Material wie beispielsweise Schichtholz, faserverstärkten Kunststoff und/oder ein elastisches Metall. Dadurch kann eine Flexibilität der Schlafoberflächenbasis erreicht werden.

Die Latten sind vorzugsweise leicht gewölbt oder profiliert ausgebildet, so dass sie unter Belastung nachgeben und den Körper des Nutzers ergonomisch unterstützen können. Alternativ kann jede Latte flach ausgebildet sein, wobei die Federwirkung durch das Material selbst oder durch eine elastische Kopplung mit der Verstelleinrichtung erreicht wird.

Die Endabschnitte der Latten sind so ausgebildet, dass sie jeweils mit einer motorischen Verstelleinrichtung gekoppelt sind. Bezogen auf eine einzelne Latte bedeutet dies, dass der erste Endabschnitt mit einer ersten Verstelleinrichtung und der zweite Endabschnitt mit einer zweiten Verstelleinrichtung gekoppelt ist. Somit ist die Vertikalposition des ersten Endabschnitts mittels der ersten Verstelleinrichtung gegenüber dem Träger verstellbar bzw. einstellbar. Weiterhin ist die Vertikalposition des zweiten Endabschnitts mittels der zweiten Verstelleinrichtung gegenüber dem Träger verstellbar bzw. einstellbar. Je Latte sind somit bevorzugt zwei Verstelleinrichtungen vorgesehen.

Die Verstelleinrichtung ist bevorzugt wirkmäßig und/oder räumlich zwischen dem Endabschnitt und dem Träger angeordnet.

Die Verstelleinrichtung ermöglicht eine gezielte Verstellung der Vertikalposition, sprich in Verstellrichtung, des mit der Verstelleinrichtung gekoppelten Endabschnitts. Bevorzugt bezeichnet die Vertikalposition die Höhenposition, welche durch eine Höhenverstellung jedes einzelnen Endabschnitts relativ zum Träger verändert werden kann. Die Vertikalposition des Endabschnitts wird in der Verstellrichtung (Höhenverstellrichtung, Vertikalrichtung) angepasst. Somit ist eine flexible Anpassung der Vertikalposition jedes einzelnen Endabschnitts ermöglicht.

Das Übersetzungsverhältnis kann auch als Wegübersetzungsverhältnis bezeichnet werden. Mit anderen Worten ist das Übersetzungsverhältnis bezogen auf den Weg das Verhältnis zwischen der resultierenden Wegstrecke (Höhenänderung), die an dem Endabschnitt der Latte erreicht wird, und der Antriebswegstrecke, die an der motorischen Verstelleinrichtung zurückgelegt wird. Das Übersetzungsverhältnis ist die resultierende Wegstrecke (Änderung der Vertikalposition der Latte, insbesondere der Höhenverstellweg und somit die Höhenänderung) je Wegstrecke, die von der Verstelleinrichtung bereitgestellt wird. Das Kraftübersetzungsverhältnis ist dabei indirekt proportional zum Wegübersetzungsverhältnis. Mit anderen Worten muss das, was an Weg "gespart" wird, als Kraft "zugesetzt" werden.

Die Hebeleinrichtung weist ein von der Vertikalposition abhängiges Übersetzungsverhältnis auf. Das Übersetzungsverhältnis ändert sich hierbei dynamisch/kontinuierlich mit der Veränderung der Vertikalposition.

Besonders bevorzugt ist die Verstelleinrichtung und insbesondere die Hebeleinrichtung, die mit dem einem Endabschnitt der Latte wirkverbunden ist, symmetrisch zu der Verstelleinrichtung und insbesondere zu deren Hebeleinrichtung, die mit dem anderen Endabschnitt der Latte wirkverbunden ist. Mit anderen Worten sind die Verstelleinrichtungen, die mit einer Latte gekoppelt sind, symmetrisch zueinander.

In einer vorteilhaften Weiterbildung ist es vorgesehen, dass die Hebeleinrichtung zwischen dem Endabschnitt der Latte und dem Träger angeordnet ist.

In einer vorteilhaften Weiterbildung weist die Vorspanneinrichtung in einer Position mit einem größeren Übersetzungsverhältnis (Wegübersetzungsverhältnis) eine höhere Vorspannung auf als in einer Position mit einem kleineren Übersetzungsverhältnis. Bei einem größeren Übersetzungsverhältnis wird mehr resultierende Wegstrecke je Antriebswegstrecke realisiert als bei einem kleineren Übersetzungsverhältnis. Dies bedeutet, dass bei dem größeren Übersetzungsverhältnis eine höhere Kraft erforderlich ist als bei dem kleineren Übersetzungsverhältnis, sofern alle anderen Rahmenbedingungen unverändert sind. Dank der Vorspanneinrichtung kann in Zuständen, in denen hohe Kräfte für die Verstellung erforderlich sind, mehr Kraft bereitgestellt werden als in Zuständen mit geringeren Kraftanforderungen. Dadurch wird die motorische Verstelleinrichtung entlastet und muss weniger Arbeit verrichten. Somit wird die Verstelleinrichtung insbesondere dann durch die Vorspanneinrichtung unterstützt, wenn die Verstellung besonders schwergängig ist. Dadurch kann diese kleiner, kompakter und kostengünstiger ausgebildet sein als im Stand der Technik.

In einer vorteilhaften Weiterbildung wirkt die Vorspanneinrichtung entgegen einer von der Schlafoberflächenbasis ausgehenden Gewichtskraft. Die Gewichtskraft, die auf die Schlafoberflächenbasis wirkt, resultiert aus den darauf liegenden Lasten, wie beispielsweise der Körpermasse einer auf der Schlafoberflächenbasis befindlichen Person oder der Eigenmasse der Latten. Die Vorspanneinrichtung stellt eine Kraft bereit, bei der zumindest eine Kraftkomponente der Gewichtskraft entgegengesetzt ist. Die motorische Verstelleinrichtung kann somit entsprechend kompakt ausgeführt werden, da sie bei einer Verstelloperation, bei der der Endabschnitt angehoben wird, durch die Vorspanneinrichtung unterstützt wird. Ein Teil der für die Verstellung erforderlichen Kraft wird durch die Vorspanneinrichtung bereitgestellt.

In einer bevorzugten Weiterbildung ist die Vorspanneinrichtung als Federelement ausgebildet. Federelemente bieten den Vorteil, dass diese einen einfachen und kompakten Aufbau aufweisen und gleichzeitig kostengünstig sind und eine hohe Lebensdauer aufweisen. Das Federelement kann insbesondere als mechanische Feder ausgeführt sein, beispielsweise in Form einer Schrauben-, Blatt-, Torsions- oder Schenkelfeder. Alternativ oder ergänzend ist es möglich, das Federelement als Gasdruckfeder zu realisieren, die durch komprimiertes Gas eine konstante oder variable Gegenkraft bereitstellt. Es kann dabei vorgesehen sein, dass die Vorspanneinrichtung eine oder mehrere Federn umfasst. Diese lassen sich in einer gewünschten Parallelschaltung oder Reihenschaltung anordnen.

Es kann bevorzugt vorgesehen sein, dass die Vorspanneinrichtung eine lineare, progressive oder degressive Federkennlinie aufweist. Somit lässt sich die Vorspanneinrichtung optimal an die Hebeleinrichtung anpassen.

In einer vorteilhaften Weiterbildung sind die Verstelleinrichtungen jeweils zwischen einer eingefahrenen Position (Vertikalposition) und einer ausgefahrenen Position (Vertikalposition) verstellbar, wobei das Übersetzungsverhältnis in der eingefahrenen Position größer ist als in der ausgefahrenen Position. Es kann dabei vorgesehen sein, dass die Hebeleinrichtung von einer geneigten Lage (eingefahrenen Position), in der sie winklig zur Verstellrichtung steht, in eine Strecklage verstellbar ist. In der Strecklage der Hebeleinrichtung wird die maximale Vertikalposition des Endabschnitts erreicht. Die maximale Vertikalposition des Endabschnitts entspricht jenem Zustand, in dem der Endabschnitt den größtmöglichen Abstand zum Träger aufweist. Der Endabschnitt hat dort den höchsten Punkt erreicht.

In der eingefahrenen Position wird ein großer resultierender Weg im Verhältnis zur Antriebswegstrecke zurückgelegt. Daher ist in dieser Position eine höhere Kraft für die Verstellung erforderlich als in der ausgefahrenen Position. In der ausgefahrenen Position wird eine kleine resultierende Wegstrecke des Endabschnitts durch eine größere Antriebswegstrecke realisiert, so dass die dafür erforderliche Kraft kleiner ist als bei einer Verstellung in der Nähe der eingefahrenen Position. Der Wechsel zwischen diesen beiden Extremlagen erfolgt bevorzugt kontinuierlich, so dass sich das Übersetzungsverhältnis (Wegübersetzungsverhältnis) bevorzugt von einem Maximum hin zu einem Minimum kontinuierlich verändert, wenn von der eingefahrenen Position in die ausgefahrene Position verstellt wird.

In einer vorteilhaften Weiterbildung weist das Übersetzungsverhältnis (Wegübersetzungsverhältnis=resultierende Wegstrecke/Antriebswegstrecke) in der ausgefahrenen Position (maximale Höhenverstellposition/Vertikalposition) einen Betrag zwischen 0,1 und 0,75 auf, besonders bevorzugt 0,25. In einer vorteilhaften Weiterbildung weist das Übersetzungsverhältnis (Wegübersetzungsverhältnis=resultierende Wegstrecke/Antriebswegstrecke) in der eingefahrenen Position (minimale Höhenverstellposition/Vertikalposition) einen Betrag zwischen 2 und 6 auf, besonders bevorzugt 4.

In einer vorteilhaften Weiterbildung sind die Verstelleinrichtungen unabhängig voneinander verstellbar. Jede der Latten ist jeweils zwei Verstelleinrichtungen zugeordnet. Jeder Endabschnitt ist mit einer der Verstelleinrichtungen gekoppelt. Somit lässt sich jeder Endabschnitt der Latten unabhängig voneinander einstellen. Dies bietet höchste Flexibilität und Anpassungsmöglichkeiten.

In einer vorteilhaften Weiterbildung kann es vorgesehen sein, dass die Verstelleinrichtungen jeweils einen motorischen Verstellantrieb aufweisen, der mit der Hebeleinrichtung wirkverbunden ist. Die von dem motorischen Verstellantrieb bereitgestellte Kraft und Verschiebung wird in die Hebeleinrichtung eingeleitet, so dass die Hebeleinrichtung eine Verlagerung des Endabschnitts herbeiführt.

Bevorzugt ist der Verstellantrieb an dem Träger festgelegt und umfasst eine Gewindespindel und eine Spindelmutter, wobei die Gewindespindel drehbar durch einen Elektromotor antreibbar ist zur translatorischen Verlagerung der auf der Gewindespindel aufgeschraubten Spindelmutter. Die Gewindespindel ist drehbar gelagert und durch einen Elektromotor antreibbar. Durch die Rotationsbewegung der Gewindespindel, die mittels des Elektromotors erzeugt wird, wird die Spindelmutter in eine definierte Richtung entlang der Längsachse der Gewindespindel bewegt. Die Spindelmutter ist mit der Hebeleinrichtung unverdrehbar um die Längsachse gekoppelt, so dass die Bewegung der Spindelmutter eine Bewegung der Hebeleinrichtung herbeiführt, die wiederum zu einer Verstellung des Endabschnitts der Latte führt. Bevorzugt ist die Spindelmutter gelenkig mit der Hebeleinrichtung gekoppelt, besonders bevorzugt gelenkig orthogonal zur Längsachse. Bevorzugt wird durch die Verlagerung der Spindelmutter die Hebeleinrichtung zwischen der geneigten Lage und der Strecklage bewegt.

In einer vorteilhaften Weiterbildung ist der Elektromotor als Schrittmotor ausgebildet. Dies bietet den Vorteil, dass die Rotation der Rotorwelle in präzise definierte Schritte unterteilt ist. Dies ermöglicht eine genaue Steuerung der Position, Geschwindigkeit und Richtung der Motorwelle, ohne dass beispielsweise ein separates Positionsfeedbacksystem wie ein Encoder erforderlich ist.

Es ist jedoch denkbar und möglich, dass der Elektromotor als Gleichstrommotor, Asynchronmotor oder Synchronmotor ausgebildet ist.

Bevorzugt ist das Gewinde zwischen der Gewindespindel und der Spindelmutter selbsthemmend ausgeführt. Dies bietet den Vorteil, dass eine ungewollte Verstellung im stromlosen Zustand des Elektromotors aufgrund der wirkenden Gewichtskraft wirksam vermieden wird. Somit kann auf eine aufwendige und gesonderte Fixiereinrichtung verzichtet werden. Ganz besonders bevorzugt weist das Gewinde der Gewindespindel bzw. der Spindelmutter einen Flankenwinkel auf, der kleiner 60° ist. In einer besonders vorteilhaften Weiterbildung sind die Spindelmutter und die Gewindespindel aus unterschiedlichen Materialien ausgebildet, wobei einer der Materialien ein Kunststoff und das andere Material ein metallischer Werkstoff ist.

In einer vorteilhaften Weiterbildung ist zwischen der Gewindespindel und dem Elektromotor ein Getriebe angeordnet. Bevorzugt ist das Getriebe als Schneckenradgetriebe, Planetengetriebe oder Wellgetriebe ausgebildet. Dies bietet den Vorteil, dass der Elektromotor kleiner und kompakter dimensioniert werden kann, da die erforderliche Leistung durch eine entsprechend hohe Drehzahl des Elektromotors realisiert wird.

In einer vorteilhaften Weiterbildung umfasst die Hebeleinrichtung eine Kniehebelanordnung. Die Kniehebelanordnung besteht aus mindestens zwei durch ein Gelenk miteinander verbundenen Hebeln, die in einer Knickbewegung zusammenwirken. Durch diese Anordnung wird eine besonders effiziente Kraftübersetzung erzielt, wobei die Kniehebelanordnung bei Annäherung an die gestreckte Endposition eine zunehmende Stabilität und eine Verringerung der notwendigen Haltekraft aufweist.

Die Kniehebelanordnung ermöglicht es, große resultierende Kräfte an den Endabschnitten bei vergleichsweise geringem Kraftaufwand durch den Verstellantrieb zu erzeugen und zugleich präzise Bewegungen durchzuführen. Dank der Kniehebelanordnung kann sowohl der Bewegungsbereich als auch die aufgebrachte Kraft an die jeweiligen Anforderungen angepasst werden. Diese Weiterbildung bietet einen robusten und effizienten Aufbau, der hohe Stabilität und präzise Bewegungssteuerung und gleichzeitig eine kompakte Anordnung bereitstellt.

In einer vorteilhaften Weiterbildung umfasst die Kniehebelanordnung einen ersten Hebel, der ein erstes Ende und ein zweites Ende aufweist, wobei das erste Ende mit dem Verstellantrieb gelenkig gekoppelt ist und das zweite Ende mit dem einen Endabschnitt der Latte gelenkig gekoppelt ist. Die Kniehebelanordnung umfasst weiterhin einen zweiten Hebel, der ein drittes Ende und ein viertes Ende aufweist, wobei das dritte Ende gelenkig mit dem Träger gekoppelt ist und das vierte Ende gelenkig mit dem ersten Hebel gekoppelt ist. Mit anderen Worten weist der zweite Hebel zwei Enden auf, die als drittes und viertes Ende bezeichnet sind, um eine Verwechselung mit den Enden des ersten Hebels zu vermeiden. Dank dieser Anordnung lässt sich ein optimales Übersetzungsverhalten sowohl für die Weg- als auch die Kraftübersetzung erzielen. Weiterhin ist eine solche Anordnung sehr stabil, insbesondere in einer Richtung orthogonal zur Vertikalrichtung/Verstellrichtung.

Das vierte Ende ist bevorzugt an einer Stelle mit dem ersten Hebel gekoppelt, die zwischen dem ersten und dem zweiten Ende des ersten Hebels liegt. Dank dieser Anordnung kann eine optimale Übersetzung der von dem Verstellantrieb in den ersten Hebel eingetragenen Kraft auf den Endabschnitt der Latte realisiert werden. Wenn sich der Endabschnitt in einer eingefahrenen Position befindet, ist der erste Hebel stark winklig zu der Verstellrichtung angeordnet. Insbesondere weist der erste Hebel einen Winkel zur Verstellrichtung zwischen 75° und 90° auf. Ebenfalls weist der zweite Hebel einen Winkel zur Verstellrichtung zwischen 75° und 90° auf. Dabei ist ein Abstand zwischen dem ersten Ende und dem dritten Ende in der eingefahrenen Position größer als in der ausgefahrenen Position. Weiterhin ist ein Abstand zwischen dem zweiten Ende und dem dritten Ende in der eingefahrenen Position kleiner als in der ausgefahrenen Position. Wenn sich der Endabschnitt in einer ausgefahrenen Position befindet, ist der erste Hebel geringfügig winklig zu der Verstellrichtung angeordnet. Insbesondere weist der erste Hebel einen Winkel zur Verstellrichtung zwischen 0° und 15° auf, was der Strecklage entspricht. Ebenfalls weist der zweite Hebel einen Winkel zur Verstellrichtung zwischen 0° und 15° auf. Versuche haben gezeigt, dass sich mit einer derartigen Anordnung sehr gute Ergebnisse in der Verstellperformance erreichen lassen, sowie gleichzeitig der Aufbau sehr stabil und bauraumsparend ist.

In einer vorteilhaften Weiterbildung ist der erste Hebel und/oder der zweite Hebel als U-förmiges Profil ausgebildet, welches ganz besonders bevorzugt aus Stahl oder einer Aluminiumlegierung gebildet ist oder eine solche umfasst. Eine solche Anordnung weist zum einen ein optimales Flächenträgheitsmoment bzw. Widerstandsmoment auf, sowie durch die vorgeschlagenen Werkstoffe eine ausreichende Festigkeit und Steifigkeit. Bevorzugt kann es vorgesehen sein, dass der erste Hebel oder/und der zweite Hebel ein oder mehrteilig ausgebildet ist bzw. sind.

Bevorzugt sind die gelenkigen Verbindungen der Hebel als Gleitlagerung ausgeführt. Für eine besonders spielfreie gelenkige Anordnung kann alternativ auch eine Wälzlagerung vorgesehen sein.

In einer vorteilhaften Weiterbildung kann es vorgesehen sein, dass sämtliche Hebeleinrichtungen des Bettgestells gleichartig ausgebildet sind oder identisch sind. Insbesondere sind die Hebeleinrichtungen, die mit dem ersten Endabschnitt und dem zweiten Endabschnitt verbunden sind, gleichartig oder identisch. Dadurch, dass die Hebeleinrichtungen auf der linken und rechten Bettseite gleichartig oder identisch sind, kann eine kostengünstige Produktion und wirtschaftliche Wartung und Pflege des Bettgestells erzielt werden.

In einer bevorzugten Weiterbildung kann es vorgesehen sein, dass das zweite Ende des ersten Hebels und der eine Endabschnitt der Latte zueinander verschiebbar gekoppelt sind zur Bereitstellung eines Längsausgleichs. Insbesondere bei einer unabhängigen Verstellbarkeit der beiden Endabschnitte einer Latte ist es zur Vermeidung von teilweise hohen Spannungen in den Komponenten erforderlich, eine Längenkompensation (Längsausgleich) durch eine gleitende Lagerung beispielsweise der Latte vorzunehmen. Zur Vermeidung von Zwangszuständen ist es zweckdienlich, wenn der erste Hebel nicht nur gelenkig an dem Endabschnitt angeschlagen ist, sondern auch in einem geringen Maße verschiebbar ist. Somit kann auf einfache Art und Weise ein Längsausgleich geschaffen werden, wenn die beiden Endabschnitte einer Latte in unterschiedlichen Vertikalpositionen zueinander sind. Dieser Fall liegt vor, wenn die Latte nicht auf beiden Seiten gleichmäßig verstellt wird, so dass sich ein Endabschnitt weiter oben befindet als der andere Endabschnitt.

In einer bevorzugten Weiterbildung ist es vorgesehen, dass der Längsausgleich unter Überwindung einer Federkraft erfolgt. Dazu ist eine Federanordnung vorgesehen, die die Latte in ihre Längsrichtung gegenüber dem ersten Hebel vorspannt. Bevorzugt ist der Längsausgleich an beiden Endabschnitten mit einer Federanordnung ausgebildet, so dass die Federanordnung an dem einen Endabschnitt entgegen der Federanordnung des anderen Endabschnitts wirkt. Somit kann eine Zentrierung der Latte bzw. eine Ausmittelung auf einfache Weise erfolgen.

Alternativ kann es vorgesehen sein, dass nur ein Endabschnitt je Latte verschiebbar mit dem ersten Hebel gekoppelt ist, während der andere Endabschnitt gelenkig und unverschiebbar an dem ihm zugeordneten ersten Hebel gehalten ist. Eine solche Anordnung kann ebenfalls eine Federanordnung umfassen.

Weiterhin ist es ebenfalls denkbar und möglich, den Längsausgleich an einer anderen Position der Hebeleinrichtung vorzusehen.

Bevorzugt kann es vorgesehen sein, dass das erste Ende mit der Spindelmutter des Verstellantriebs gelenkig gekoppelt ist. Dazu kann es vorgesehen sein, dass die Spindelmutter zwei bolzenartige Vorsprünge aufweist, die in einer Ausnehmung des ersten Endes aufgenommen sind. Insbesondere kann es vorgesehen sein, dass die Vorsprünge einen Zweiflach aufweisen, wobei die Ausnehmung ein seitlich offenes Loch darstellt, so dass die Spindelmutter durch den Zweiflach in das offene Loch eingeführt werden kann und durch Verdrehen in diesem gesichert aufgenommen ist. Eine derartige Kopplung funktioniert wie ein Bajonettverschluss. Dank dieser Ausführung kann die Anordnung einfach montiert werden.

In einer vorteilhaften Weiterbildung umfasst die Vorspanneinrichtung eine Schenkelfeder mit zwei Schenkeln, wobei der eine Schenkel mit dem zweiten Ende gekoppelt ist und der andere Schenkel mit dem dritten Ende gekoppelt ist. Die Schenkelfeder weist dabei bevorzugt einen Abschnitt mit zumindest einer Windung zwischen den Schenkeln auf. Alternativ oder zusätzlich kann die Vorspanneinrichtung eine Schraubenfeder umfassen, die mit dem ersten Ende und dem dritten Ende gekoppelt ist. Die Schraubenfeder ist bevorzugt eine Zugfeder. Die genannten Anordnungen haben sich als besonders kompakt und robust erwiesen, die kostengünstig sind und einfach montiert werden können.

In einer vorteilhaften Weiterbildung ist eine Steuereinheit vorgesehen, die mit den Verstelleinrichtungen wirkverbunden ist. Die Steuereinheit ist derart ausgebildet, die Verstelleinrichtungen, insbesondere die Verstellantriebe bzw. deren Elektromotoren, elektrisch anzusteuern bzw. zu regeln. Dies kann beispielsweise mittels Pulsweitenmodulation erfolgen. Dazu kann es vorgesehen sein, dass die Steuereinheit zumindest einen Microcontroller und eine Halbbrückenschaltung mit MOSFETs umfasst. Die Steuereinheit ist mit einer Spannungsquelle verbindbar. Die Spannungsquelle kann durch ein Versorgungsnetz in einem Gebäude oder durch einen Akkumulator bereitgestellt werden.

Ganz besonders bevorzugt ist die Steuereinheit als zentrale Steuereinheit ausgebildet, die mit sämtlichen Verstelleinrichtungen verbunden ist. Insbesondere bietet sich hier ein Bus-System an, welches zur einfachen Verbindung der Verstelleinrichtungen mit der Steuereinheit dient. Hierzu kann beispielsweise ein CAN-Bus oder LIN-Bus zum Einsatz kommen.

In einer vorteilhaften Weiterbildung weist die Steuereinheit ein drahtloses Datenübertragungssystem auf. Dank dieses Datensystems werden die in der Steuereinheit gespeicherten Daten, wie beispielsweise die Vertikalposition oder die ermittelten Kräfte, an eine externe Datenverarbeitungseinrichtung, etwa einen Server oder ein Smartphone, weitergeleitet, wodurch eine Fernüberwachung des Bettgestells ermöglicht wird.

In einer vorteilhaften Weiterbildung sind die Endabschnitte der Latten jeweils mit einer jeweiligen Kraftmesseinrichtung wirkverbunden. In anderen Worten ist je Endabschnitt eine Kraftmesseinrichtung vorgesehen. Diese kann beispielsweise als Kraftmessdose ausgebildet sein. Dies bietet den Vorteil, dass die auf jeden einzelnen Endabschnitt wirkende Kraft voneinander losgelöst bestimmt werden kann, so dass eine genaue Bestimmung der Kraftverteilung ermöglicht ist. Dadurch kann auf eine Liegeposition einer sich auf der Schlafoberflächenbasis befindlichen Person geschlossen werden.

Bevorzugt ist es vorgesehen, dass die Kraftmesseinrichtung zwischen der Verstelleinrichtung und dem Träger angeordnet ist, insbesondere ist diese direkt unterhalb der Hebelanordnung angeordnet, so dass die auf den Endabschnitt wirkende Kraft direkt über die Hebelanordnung in die Kraftmesseinrichtung eingeleitet wird. Dies bietet den Vorteil, dass eine Messung mit senkrechter Krafteinwirkung unabhängig von der jeweiligen Position der darüberliegenden Verstelleinrichtung möglich ist und somit die Messgenauigkeit erhöht werden kann, so dass die im Endabschnitt wirkende Kraft sicher und zuverlässig ermittelbar ist.

In einer vorteilhaften Weiterbildung kann eine Kamera zur Aufzeichnung einer auf dem Bettgestell liegenden bzw. befindlichen Person vorgesehen sein. Die Kamera ist bevorzugt so angeordnet, dass diese die gesamte Schlafoberflächenbasis aufzeichnen kann. Bevorzugt kann die Kamera als optische Kamera und/oder Infrarotkamera ausgebildet sein.

Weiterhin kann alternativ statt einer Kamera auch ein Radargerät unter Verwendung elektromagnetischer Wellen verwendet werden. Mittels dieses Systems ist nicht nur die Positionserkennung der Person möglich, sondern auch die Ermittlung des Schlafverhaltens und des Pulses basierend auf den Bewegungen der auf dem Bettgestell liegenden Person. Weiterhin bietet die Verwendung eines Radargeräts den Vorteil, dass die Datenschutzbestimmungen leichter eingehalten werden können, da keine Videoaufnahmen angefertigt werden.

In einer vorteilhaften Weiterbildung kann es vorgesehen sein, dass die Kraftmesseinrichtungen und/oder die Kamera und/oder das Radargerät mit der Steuereinheit wirkverbunden sind. Die Steuereinheit ist dabei so eingerichtet, dass diese die von den Kraftmesseinrichtungen und/oder der Kamera und/oder des Radargeräts bereitgestellten Daten auswertet und daraus resultierend die Verstelleinrichtungen ansteuert.

In einer vorteilhaften Weiterbildung ist es vorgesehen, dass die Verstelleinrichtung ein Mittel zur Bestimmung der Vertikalposition aufweist, das bevorzugt mit der Steuereinheit verbunden ist. Dies kann durch einen Absolutpositionssensor erfolgen. Alternativ oder zusätzlich kann es vorgesehen sein, dass der Elektromotor einen Hallsensor umfasst, so dass die Umdrehungsanzahl der Rotorwelle mittels sogenannter Hallcounts bestimmt werden kann. Aufgrund der bekannten Kinematik der Verstelleinrichtung kann die Steuereinheit, die mit dem Hallsensor verbunden ist, die Vertikalposition errechnen.

In einer bevorzugten Weiterbildung ist es vorgesehen, dass die Steuereinheit eine zentrale Steuereinheit ist, die derart eingerichtet ist, die Vertikalpositionen der Verstelleinrichtungen und/oder die von den Kraftmesseinrichtungen gemessenen Kräfte zu erfassen und daraus resultierend die Vertikalposition durch Ansteuerung der Verstelleinrichtungen so anzupassen, dass dadurch eine auf der Schlafoberflächenbasis liegende bzw. befindliche Person umgelagert wird oder eine Umlagerung unterstützt wird. Das System ermöglicht einen 24 Stunden/sieben Tage die Woche (24/7) Pflegeservice, wobei eine von der Pflegemedizin geforderte permanente Mikroverlagerung als auch eine Mobilisierung der liegenden Person durch größere Bewegungsabläufe der gesamten Liegefläche ermöglicht ist. Dank dieser Weiterbildung kann das Auftreten von Dekubitus bei der Person wirksam vermieden oder zumindest deutlich reduziert werden.

In einer weiteren vorteilhaften Weiterbildung ist es vorgesehen, dass der Träger zumindest mit einem Rad verbunden ist. Besonders bevorzugt ist der Träger mit drei oder vier Rädern verbunden, wobei zumindest eines der Räder mit einer Bremseinrichtung versehen ist. Dank des Rades bzw. der Räder kann das Bettgestell einfach von einem Ort zu einem anderen Ort bewegt werden, ohne dass hohe Kräfte dafür erforderlich sind.

Weiterhin wird zur Lösung der Aufgabe ein Pflegebett vorgeschlagen, umfassend ein Bettgestell nach einem der zuvor genannten Aspekte. Bevorzugt weist das Pflegebett eine Matratze auf, die auf der Schlafoberflächenbasis angeordnet ist.

Weiterhin wird zur Lösung der oben genannten Aufgabe ein Verfahren zum Betreiben eines Pflegebetts vorgeschlagen, wobei das Pflegebett ein Bettgestell nach einem der zuvor genannten Ausführungsvarianten umfasst. Erfindungsgemäß werden die Vertikalpositionen der Verstelleinrichtungen und/oder die von den Kraftmesseinrichtungen gemessenen Kräfte von der Steuereinheit erfasst und daraus resultierend die Vertikalposition durch Ansteuerung der Verstelleinrichtungen so angepasst, so dass dadurch eine im Pflegebett liegende bzw. befindliche Person umgelagert wird oder eine Umlagerung unterstützt wird. Dank dieser Weiterbildung kann das Auftreten von Dekubitus bei der Person wirksam vermieden oder zumindest deutlich reduziert werden.

Weiterhin wird zur Lösung der oben genannten Aufgabe ein Verfahren zum Betreiben eines Pflegebetts vorgeschlagen, wobei das Pflegebett ein Bettgestell nach einem der zuvor genannten Ausführungsvarianten umfasst. Erfindungsgemäß werden mittels der Steuereinheit die gemessenen Kräfte der Kraftmesseinrichtungen ausgewertet und daraus ein Kraftverteilungsbild erzeugt zur Darstellung der Liegeposition einer im Pflegebett liegenden Person. Die Steuereinheit wertet dabei die gemessenen Kräfte derart aus, dass daraus ein Kraftverteilungsbild erzeugt wird. Aus diesem Kraftverteilungsbild bestimmt bzw. schließt die Steuereinheit auf die Liegeposition der im Pflegebett liegenden bzw. befindlichen Person, so dass daraus frühzeitig erkannt werden kann, wenn diese Liegeposition über längere Zeit konstant ist und somit diese verändert werden sollte, um dem Auftreten von Dekubitus entgegenzuwirken.

Das erfindungsgemäße Bettgestell bzw. das erfindungsgemäße Pflegebett bildet bevorzugt einen Pflegeroboter in Form eines Bettes, der autonom oder teilautonom nach Sensorinformationen (Kraftmesseinrichtung, Positionssensor) und Algorithmen (Verfahren zum Betreiben des Pflegebettes und anderer Regelverfahren) anstelle eines Menschen bestimmte Pflegetätigkeiten verrichten kann. Derartige Pflegetätigkeiten wären beispielsweise die Überwachung und die laufende Druckentlastung einer auf dem Bettgestell bzw. dem Pflegebett liegenden Person.

In einer vorteilhaften Weiterbildung des Verfahrens kann es vorgesehen sein, dass mittels der Steuereinheit eine von der Kamera erfasste Aufnahme ausgewertet wird und daraus die Liegeposition einer im Pflegebett liegenden Person ermittelt wird. Dies bietet den Vorteil, dass zwei unabhängige Messprinzipien zum Einsatz kommen, so dass dadurch die Betriebssicherheit des Pflegebetts erhöht ist.

Weiterhin wird die genannte Aufgabe durch ein Verfahren zur Kalibrierung eines Pflegebetts gelöst, wobei das Pflegebett ein Bettgestell nach einem der zuvor genannten Ausführungsvarianten umfasst. Erfindungsgemäß werden mittels der Steuereinheit die gemessenen Kräfte der Kraftmesseinrichtungen erfasst, wobei mittels der Steuereinheit eine von der Kamera erfasste Aufnahme ausgewertet wird und daraus die Liegeposition einer im Pflegebett liegenden Person ermittelt wird, so dass die Steuereinheit unter Nutzung eines neuronalen Netzwerks die gemessenen Kräfte und die mittels der Kamera ermittelte Liegeposition korreliert, so dass im Anschluss lediglich aus den gemessenen Kräften die Liegeposition wirksam bestimmt werden kann. Mit anderen Worten wird eine Korrelation zwischen den gemessenen Kräften und den von der Kamera ermittelten Liegepositionen erstellt und die Steuereinheit derart trainiert, dass diese auch ohne die Daten der Kamera zuverlässig auf die Liegeposition schließen kann. Dank des Kalibrierungsverfahrens kann die Genauigkeit der Bestimmung der Liegeposition basierend auf den Kraftmessungen deutlich erhöht werden, so dass auf einen dauerhaften Einsatz der Kamera zur Bestimmung der Liegeposition verzichtet werden kann. Dies bietet den Vorteil, dass keine datenschutzrelevanten Informationen durch das Pflegebett ermittelt werden, so dass der Verwaltungsaufwand für das Betreiben eines solchen Pflegebetts deutlich reduziert ist.

Ein neuronales Netzwerk ist dazu ausgebildet, durch einen Trainingsprozess die Gewichtungsparameter anzupassen, um eine spezifizierte Abbildungs- oder Entscheidungsfunktion zu erlernen. Der Trainingsprozess erfolgt üblicherweise durch die Minimierung eines vorgegebenen Fehlermaßes, z. B. unter Anwendung eines Backpropagation-Algorithmus oder einer vergleichbaren Optimierungsmethode.

Es wird darauf hingewiesen, dass die Merkmale der angegebenen Weiterbildungen und vorteilhaften Ausgestaltungen im Rahmen des technisch Möglichen frei miteinander kombiniert werden können, auch wenn dieses im Text nicht explizit angegeben ist. Dies gilt insbesondere auch über die Grenzen der Anspruchskategorien Vorrichtung und Verfahren hinweg.

Weitere Vorteile und Merkmale des erfindungsgemäßen Bettgestelles bzw. Pflegebetts ergibt sich aus den nachfolgenden Ausführungsbeispielen, die anhand der Figuren (Figur = Fig.) näher erläutert werden.

In diesen zeigen:
- Fig. 1:: eine schematisierte Ansicht eines erfindungsgemäßen Pflegebetts mit einem erfindungsgemäßen Bettgestell mit einer Latte in einer ersten Einstellung,
- Fig. 2:: eine weitere schematisierte Ansicht des erfindungsgemäßen Bettgestells in einer zweiten Einstellung,
- Fig. 3:: eine schematisierte Detailansicht einer Verstelleinrichtung des Bettgestells in einer ausgefahrenen Position,
- Fig. 4:: eine schematisierte Detailansicht der Verstelleinrichtung des Bettgestells in einer eingefahrenen Position,
- Fig. 5:: ein Wege-Diagramm einer Hebeleinrichtung des erfindungsgemäßen Bettgestells,
- Fig. 6:: eine schematisierte Seitenansicht des erfindungsgemäßen Bettgestells, und
- Fig. 7:: eine schematisierte Detailansicht eines Längsausgleich des erfindungsgemäßen Bettgestells.

In den verschiedenen Figuren sind gleiche Teile stets mit den gleichen Bezugszeichen versehen und werden daher in der Regel auch jeweils nur einmal benannt bzw. erwähnt.

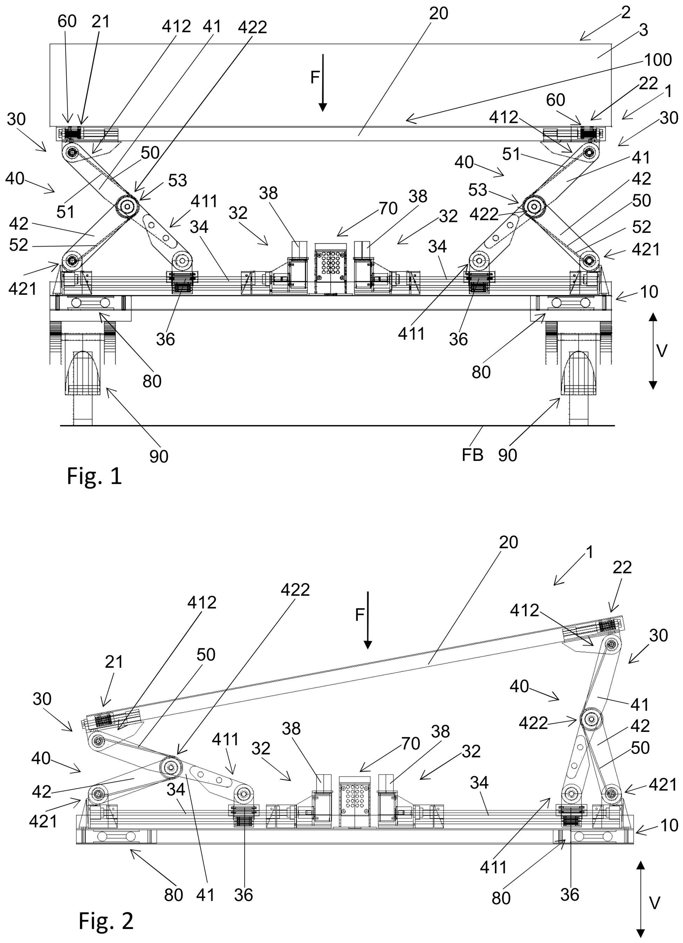

Fig. 1 zeigt eine schematisierte Ansicht eines Pflegebetts 2 mit einem erfindungsgemäßen Bettgestell 1 und einer Matratze 3.

Das Bettgestell 1 umfasst einen als Rahmen ausgebildeten Träger 10 und eine Mehrzahl von Latten 20. Die Latten 20 weisen jeweils zwei Endabschnitte 21, 22, auf, wobei die Latten 20 eine Schlafoberflächenbasis 100 ausbilden. Die Endabschnitte 21, 22 können auch als erster Endabschnitt 21 und zweiter Endabschnitt 22 bezeichnet werden. Die Endabschnitte 21, 22 sind jeweils mittels einer jeweiligen motorischen Verstelleinrichtung 30 mit dem Träger 10 verbunden, so dass die Endabschnitte 21, 22 gegenüber dem Träger 10 in ihrer Vertikalposition verstellbar sind. Erfindungsgemäß umfasst jeder der Verstelleinrichtungen 30 eine Hebeleinrichtung 40 mit einem von der Vertikalposition abhängigen Übersetzungsverhältnis und eine Vorspanneinrichtung 50. Die Vorspanneinrichtung 50 spannt die Hebeleinrichtung 40 in eine Verstellrichtung V vor.

Die Verstelleinrichtungen 30 sind jeweils zwischen einer eingefahrenen Position und einer ausgefahrenen Position verstellbar, wobei das Übersetzungsverhältnis in der eingefahrenen Position größer ist als in der ausgefahrenen Position.

In der in Fig. 1 dargestellten Einstellung der beiden Endabschnitte 21, 22 sind diese in der gleichen Vertikalposition (Höhenverstellposition) und somit ist die Latte 20 im Wesentlichen parallel zu einer Oberfläche FB, auch als Fußboden bezeichnet. Auf der Oberfläche FB stehen die mit dem Träger 10 verbundenen Räder 90. Der Träger 10 ist mit in Summe vier Rädern verbunden, wobei zwei der vier Räder 90 drehbar um eine Achse an dem Träger 10 befestigt sind. Die Achse ist parallel zu einer Normalrichtung der Oberfläche FB. Die Verstellrichtung V ist ebenfalls parallel zu der Normalenrichtung der Oberfläche FB. Die Normalenrichtung ist orthogonal zu der Ebene, die die Oberfläche FB bildet.

Die beiden Endabschnitte 21, 22 in der Fig. 1 befinden sich in einer Vertikalposition zwischen einer eingefahrenen Position, in der der jeweilige Endabschnitt 21, 22 am nächsten zur Oberfläche FB angeordnet ist, und einer ausgefahrenen Position, in der der jeweilige Endabschnitt 21, 22 am weitesten von der Oberfläche FB entfernt ist.

Die Verstelleinrichtungen 30 sind über die Hebeleinrichtungen 40 mit den Endabschnitten 21, 22 gekoppelt, so dass die Verstelleinrichtung 30 dafür sorgt, dass die Endabschnitte 21, 22 zwischen der eingefahrenen Position und der ausgefahrenen Position verstellbar sind. Dabei ist das Übersetzungsverhältnis (Wegübersetzungsverhältnis) der Hebeleinrichtung 40 in der eingefahrenen Position größer als in der ausgefahrenen Position. Wie dies realisiert ist, wird weiter unten noch genauer ausgeführt.

Grundsätzlich sind alle Verstelleinrichtungen 30 des Bettgestells, sprich zwei pro Latte 20, unabhängig voneinander verstellbar. Die Verstelleinrichtungen 30 weisen jeweils einen motorischen Verstellantrieb 32 auf, der an dem Träger 10 angebracht ist. Der Verstellantrieb 32 ist als Drehspindelantrieb ausgeführt und weist eine durch einen als Schrittmotor ausgebildeten Elektromotor 38 antreibbare Gewindespindel 34 auf, die in eine Spindelmutter 36 eingeschraubt ist. Zwischen dem Elektromotor 38 und der Gewindespindel 34 ist ein Schneckengetriebe angeordnet, wobei die Rotorwelle des Elektromotors 38 mit einer Schnecke und die Gewindespindel 34 mit dem Schneckenrad drehfest gekoppelt ist. Die Schnecke greift in das Schneckenrad ein. Die Gewindespindel 34 ist an ihrem freien Ende drehbar am Träger 10 gelagert. Dadurch kann diese optimal abgestützt werden.

Die Hebeleinrichtung 40 ist als Kniehebelanordnung ausgeführt und auf beiden Seiten entsprechend symmetrisch ausgestaltet. Insgesamt sind die beiden Verstelleinrichtungen 30 einer Latte 20 zueinander symmetrisch ausgebildet. Die Kniehebelanordnung weist einen ersten Hebel 41 auf, der ein erstes Ende 411 und ein zweites Ende 412 umfasst. Der erste Hebel 41 ist dabei als U-förmiges Stahlprofil ausgebildet, wobei das erste Ende 411 mit der Spindelmutter 36 des Verstellantriebs 32 gelenkig gekoppelt ist. Dies erfolgt durch eine Bolzenverbindung, wobei die Spindelmutter 32 mit einem Joch gekoppelt ist, welches das erste Ende 411 aufnimmt. Ein Bolzen erstreckt sich durch das Joch und das erste Ende 411 zur Bereitstellung der gelenkigen Verbindung. Das zweite Ende 412 ist mit dem einen Endabschnitt 21, 22 der Latte 20 gelenkig gekoppelt. Dabei ist der Endabschnitt 21, 22 der Latte über einen Längsausgleich 60 mit dem zweiten Ende 412 gekoppelt. Der Längsausgleich 60 weist ein U-förmiges Stanzbiegeteil auf, das mit dem zweiten Ende 412 gelenkig mittels einer Bolzenverbindung gekoppelt ist. Weiterhin weist die Kniehebelanordnung einen zweiten Hebel 42 auf. Der zweite Hebel 42 ist dabei ungefähr halb so lang wie der erste Hebel 41. Der zweite Hebel 42 umfasst ein drittes Ende 421 und ein viertes Ende 422, wobei das dritte Ende 421 gelenkig mit dem Träger 10 gekoppelt ist. Dazu ist wieder eine Bolzenverbindung vorgesehen, die eine entsprechende drehbare Lagerung des zweiten Hebels 42 an dem Träger 10 bereitstellt. Das vierte Ende 422 ist gelenkig mittels einer Bolzenverbindung mit dem ersten Hebel 41 gekoppelt, wobei das vierte Ende 422 ungefähr auf der halben Strecke zwischen dem ersten und zweiten Ende 411, 412 gelenkig an dem ersten Hebel 41 angeschlagen ist. Auch der zweite Hebel 42 ist wie der erste Hebel 41 als U-förmiges Stahlbauteil ausgeführt.

Die Vorspanneinrichtung 50 weist in einer Position mit einem größeren Übersetzungsverhältnis eine höhere Vorspannung auf als in einer Position mit einem kleineren Übersetzungsverhältnis, wobei die Vorspanneinrichtung 50 entgegen einer von der Schlafoberflächenbasis 100 ausgehenden Gewichtskraft F wirkt. Konkret ist die Vorspanneinrichtung 50 als eine Schenkelfeder ausgeführt, die zwei Schenkel 51, 52 umfasst, wobei zwischen den Schenkeln 51, 52 ein Windungsabschnitt 53 vorgesehen ist. Der erste Schenkel 51 ist mit dem zweiten Ende 412 gekoppelt, in dem der erste Schenkel 51 ein hakenförmiges Ende aufweist, das den Bolzen umgreift, der sich durch das zweite Ende 412 hindurch erstreckt. Der zweite Schenkel 52 ist mit dem dritten Ende 421 gekoppelt, in dem auch dieser Schenkel 52 ein hakenförmiges Ende aufweist, das den Bolzen umgreift, der sich durch das dritte Ende 421 erstreckt. Der Windungsabschnitt 53 umschlingt die gelenkige Verbindung zwischen dem ersten und zweiten Hebel 41, 42. Die Vorspanneinrichtung 50 wirkt dabei derart auf die Kniehebelanordnung, dass diese die Kniehebelanordnung in Verstellrichtung vorspannt. Die Vorspannung wirkt dabei so, dass diese den Endabschnitt nach oben drückt.

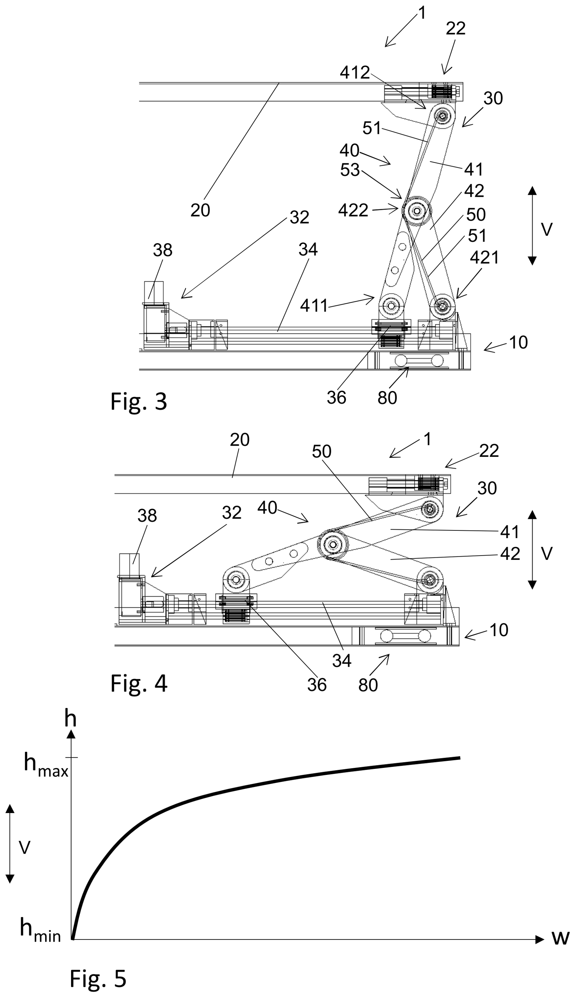

Die als Kniehebelanordnung ausgeführte Hebeleinrichtung 40 kann von einer geneigten Lage (eingefahrene Position) in eine Strecklage verstellt werden, so dass eine maximale Vertikalposition des Endabschnitts (ausgefahrene Position) in der Strecklage der Hebeleinrichtung 40 erreicht wird. In der Fig. 2 lässt sich dies besonders gut erkennen. Die Fig. 2 zeigt die Verstelleinrichtung 30 auf der linken Seite in der eingefahrenen Position. Die Verstelleinrichtung 30 auf der rechten Seite ist in der ausgefahrenen Position. Weiterhin ist in der Fig. 3 und Fig. 4 jeweils eine Detailansicht der Verstelleinrichtung 30 in diesen beiden Positionen dargestellt. Dabei ist in der Fig. 3 die ausgefahrene Position (Vertikalposition) und in der Fig. 4 die eingefahrene Position (Vertikalposition) dargestellt.

Die Hebeleinrichtung 40 ist so eingerichtet, dass in der eingefahrenen Position ein großer resultierender Weg (Vertikalpositionsänderung, Höhenverstellweg) im Verhältnis zur Antriebswegstrecke zurückgelegt wird. Die Antriebsstrecke ist der Weg, den die Spindelmutter 36 zurücklegt. Somit ist in der eingefahrenen Position eine höhere Kraft für die Verstellung erforderlich als in der bzw. kurz vor der ausgefahrenen Position. In der ausgefahrenen Position wird im Vergleich zur eingefahrenen Position eine kleine resultierende Wegstrecke (Vertikalpositionsänderung, Höhenverstellweg) des Endabschnitts durch eine größere Antriebswegstrecke realisiert, so dass die erforderliche Kraft im Vergleich zu der eingefahrenen Position reduziert ist. Der Elektromotor 38 muss somit weniger Arbeit verrichten. Der Wechsel zwischen diesen beiden Extremlagen erfolgt kontinuierlich, so dass sich das Übersetzungsverhältnis (Wegübersetzungsverhältnis) von einem Maximum hin zu einem Minimum kontinuierlich verändert, wenn von der eingefahrenen Position in die ausgefahrene Position verstellt wird. In der Fig. 5 ist zur verbesserten Verdeutlichung ein Wege-Diagramm dargestellt. Dabei ist auf der Abszissenachse der Weg w der Spindelmutter 36 bzw. des ersten Endes 411 und auf der Ordinatenachse die Höhe h des Endabschnitts (=Vertikalposition) aufgetragen, wobei in der eingefahrenen Position die minimale Höhe hₘᵢₙ und in der ausgefahrenen Position die maximale Höhe hₘₐₓ vorliegt. Wenn eine Verstellung von minimaler Höhe zu maximaler Höhe erfolgt, zeigt das Diagramm deutlich, dass zu Beginn mit einer geringen Wegänderung Δw relativ viel Höhenänderung Δh erzielt wird. Am Ende, also kurz vor Erreichung von hₘₐₓ ist es genau umgekehrt. Die Vorspannung der Vorspanneinrichtung 50 ist bei minimaler Höhe hₘᵢₙ größer als bei maximaler Höhe hₘₐₓ, da somit die wirkende Übersetzung ausgeglichen wird und der Elektromotor 38 bei der Verstellung unterstützt wird.

Dank der Vorspanneinrichtung 50 kann eine hohe Vorspannkraft bereitgestellt werden, wenn diese aufgrund der Hebelbedingungen der Kniehebelanordnung benötigt wird. Die Vorspanneinrichtung 50 kompensiert zumindest teilweise die Einflüsse des sich verändernden Übersetzungsverhältnisses. Somit wird dank der Vorspanneinrichtung 50 eine optimale Unterstützung des Verstellantriebs 32 erreicht.

Auf dem Träger 10 ist eine Steuereinheit 70 vorgesehen, die als zentrale Steuereinheit ausgebildet ist und mit sämtlichen Verstellantrieben 32 elektrisch über ein Bus-System verbunden ist. Die Steuereinheit 70 steuert die Verstellantriebe 32 elektrisch an und ist mit einer Spannungsquelle verbunden.

Weiterhin sind Kraftmesseinrichtungen 80 in Form von Kraftmessdosen zwischen der Verstelleinrichtung 30 und dem Träger 10 angeordnet, so dass je Latte 20 zwei Kraftmesseinrichtungen 80 vorgesehen sind, die jeweils die von dem Endabschnitt 21, 22 ausgehende Kraft ermitteln. Dabei wirkt die Kraft senkrecht auf die Kraftmesseinrichtungen 80, so dass eine hohe Messgenauigkeit erreicht wird. Die Kraftmesseinrichtung 80 ist über eine Datenleitung mit der Steuereinheit 70 verbunden, wobei die Steuereinheit 70 die von der Kraftmesseinrichtung ermittelten Daten weiterverarbeitet.

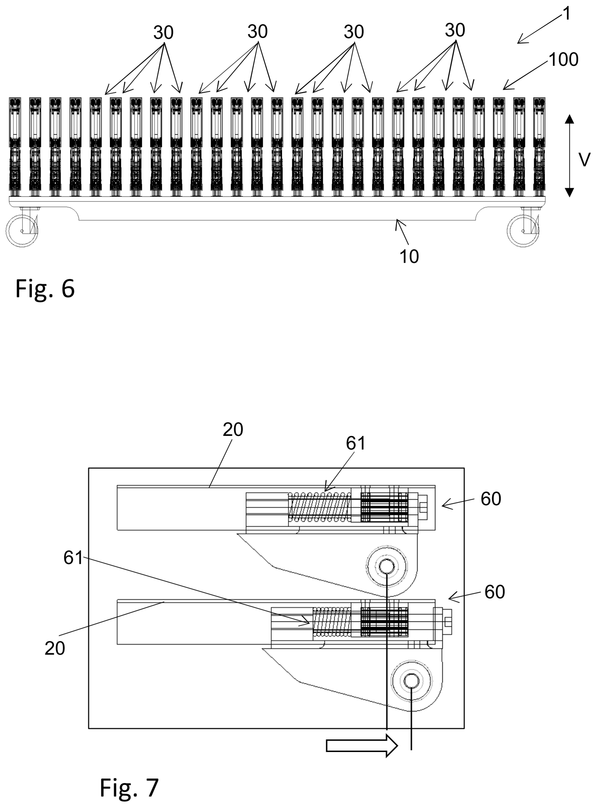

Fig. 6 zeigt eine schematisierte Seitenansicht des erfindungsgemäßen Bettgestells 1, wobei der Träger 10 mit einer Vielzahl von Verstelleinrichtungen 30 versehen ist. Die Latten 20 werden von den Verstelleinrichtungen 30 gelagert, wobei sich alle Verstelleinrichtungen 30 in einer gleichen Vertikalposition befinden und somit eine ebene Schlafoberflächenbasis 100 bilden. Alle Verstelleinrichtungen 30 weisen den gleichen Aufbau auf und sind mit der zentralen Steuereinheit 70 verbunden. Jede Verstelleinrichtung 30 ist eine Kraftmesseinrichtung 80 zugeordnet, die alle samt wiederum mit der Steuereinheit 70 verbunden sind.

In der Fig. 7 ist ein Längenausgleich 60 schematisiert dargestellt, wobei zwischen der oberen Darstellung und der unteren Darstellung der Fig. 7 eine Längenausgleichsbewegung realisiert wurde. Der Längenausgleich 60 ist ein verschiebbarer Schlittenmechanismus und zwischen dem Endabschnitt 21,22 der Latte 20 und dem zweiten Ende 412 des ersten Hebels 41 angeordnet. Der Längenausgleich 60 weist dabei eine als Druckfeder ausgebildete Spiralfeder 61 auf, so dass hier eine Vorspannung in Richtung der Erstreckungsrichtung (Längsrichtung) der Latte 20 vorgesehen ist. Damit kann vermieden werden, dass der Längsausgleich 60 ein Spiel aufweist. Weiterhin wird eine Zentrierung der Latte 20 erreicht, wenn beide Längsausgleiche 60 einer Latte 20 gegensinnig vorgespannt sind.

### Bezugszeichenliste

- 1: Bettgestell
- 2: Pflegebett
- 3: Matratze
- 10: Träger
- 20: Latte
- 21: erste Endabschnitt
- 22: zweiter Endabschnitt
- 30: motorische Verstelleinrichtung
- 32: Verstellantrieb
- 34: Gewindespindel
- 36: Spindelmutter
- 38: Elektromotor
- 40: Hebeleinrichtung
- 41: erster Hebel
- 411: erstes Ende
- 412: zweites Ende
- 42: zweiter Hebel
- 421: drittes Ende
- 422: viertes Ende
- 50: Vorspanneinrichtung
- 51: erste Schenkel
- 52: zweiter Schenkel
- 53: Windungsabschnitt
- 60: Längsausgleich
- 61: Spiralfeder
- 70: Steuereinheit
- 80: Kraftmesseinrichtung
- 90: Rad
- 100: Schlafoberflächenbasis
- V: Verstellrichtung
- F: Gewichtskraft
- FB: Oberfläche (Fußboden)

## Patentansprüche

1. Bettgestell (1) für ein Pflegebett (2), umfassend einen Träger (10) und eine Mehrzahl von Latten (20), die jeweils zwei Endabschnitte (21, 22) aufweisen und zur Ausbildung einer Schlafoberflächenbasis (100) eingerichtet sind, wobei die Endabschnitte (21, 22) jeweils mittels einer jeweiligen motorischen Verstelleinrichtung (30) gegenüber dem Träger (10) in ihrer Vertikalposition verstellbar sind, **dadurch gekennzeichnet, dass** jeder der Verstelleinrichtungen (30) eine Hebeleinrichtung (40) mit einem von der Vertikalposition abhängigen Übersetzungsverhältnis und eine Vorspanneinrichtung (50) aufweist, wobei die Vorspanneinrichtung (50) die Hebeleinrichtung (40) in eine Verstellrichtung (V) vorspannt.

2. Bettgestell (1) nach Anspruch 1, **dadurch gekennzeichnet, dass** die Vorspanneinrichtung (50) in einer Position mit einem größeren Übersetzungsverhältnis eine höhere Vorspannung aufweist als in einer Position mit einem kleineren Übersetzungsverhältnis.

3. Bettgestell (1) nach einem der vorangehenden Ansprüche, **dadurch gekennzeichnet, dass** die Vorspanneinrichtung (50) entgegen einer von der Schlafoberflächenbasis (100) ausgehenden Gewichtskraft (F) wirkt.

4. Bettgestell (1) nach einem der vorangehenden Ansprüche, **dadurch gekennzeichnet, dass** die Vorspanneinrichtung (50) als Federelement ausgebildet ist.

5. Bettgestell (1) nach einem der vorangehenden Ansprüche, **dadurch gekennzeichnet, dass** die Verstelleinrichtungen (30) jeweils zwischen einer eingefahrenen Position und einer ausgefahrenen Position verstellbar ist, wobei das Übersetzungsverhältnis in der eingefahrenen Position größer ist als in der ausgefahrenen Position.

6. Bettgestell (1) nach einem der vorangehenden Ansprüche, **dadurch gekennzeichnet, dass** die Verstelleinrichtungen (30) unabhängig voneinander verstellbar sind.

7. Bettgestell (1) nach einem der vorangehenden Ansprüche, **dadurch gekennzeichnet, dass** die Verstelleinrichtungen (30) jeweils einen motorischen Verstellantrieb (32) aufweisen, der mit der Hebeleinrichtung (41, 42) wirkverbunden ist.

8. Bettgestell (1) nach einem der vorangehenden Ansprüche, **dadurch gekennzeichnet, dass** die Hebeleinrichtung (40) eine Kniehebelanordnung umfasst, wobei bevorzugt die Kniehebelanordnung einen ersten Hebel (41) umfasst, der ein erstes Ende (411) und ein zweites Ende (412) aufweist, wobei das erste Ende (411) mit dem Verstellantrieb (32) gelenkig gekoppelt ist und das zweite Ende (412) mit dem einen Endabschnitt (21, 22) der Latte (20) gelenkig gekoppelt ist, und dass die Kniehebelanordnung einen zweiten Hebel (42) umfasst, der ein drittes Ende (421) und ein viertes Ende (422) aufweist, wobei das dritte Ende (421) gelenkig mit dem Träger (10) gekoppelt ist und das vierte Ende (422) gelenkig mit dem ersten Hebel (41) gekoppelt ist, wobei besonders bevorzugt das zweite Ende (412) des ersten Hebels (41) und der eine Endabschnitt (21, 22) der Latte (20) zueinander verschiebbar gekoppelt sind zur Bereitstellung eines Längsausgleichs (60).

9. Bettgestell nach Anspruch 8, **dadurch gekennzeichnet, dass** die Vorspanneinrichtung (50) eine Schenkelfeder mit zwei Schenkel (51, 52) umfasst, wobei der eine Schenkel (51) mit dem zweiten Ende (412) gekoppelt ist und der andere Schenkel (52) mit dem dritten Ende (421) gekoppelt ist, und/oder dass die Vorspanneinrichtung (50) eine Schraubenfeder umfasst, die mit dem ersten Ende (412) und dem dritten Ende (421) gekoppelt ist.

10. Bettgestell nach einem der vorangehenden Ansprüche, **dadurch gekennzeichnet, dass** eine Steuereinheit (70) vorgesehen ist, die mit den Verstelleinrichtungen (30) wirkverbunden ist.

11. Bettgestell (1) nach einem der vorangehenden Ansprüche, **dadurch gekennzeichnet, dass** die Endabschnitte (21, 22) der Latten (20) jeweils mit einer jeweiligen Kraftmesseinrichtung (80) wirkverbunden sind, wobei bevorzugt die Kraftmesseinrichtung (80) zwischen der Verstelleinrichtung (30) und dem Träger (10) angeordnet ist.

12. Bettgestell (1) nach einem der vorangehenden Ansprüche, **dadurch gekennzeichnet, dass** eine Kamera vorgesehen ist zur Aufzeichnung einer auf dem Bettgestell liegenden Person.

13. Bettgestell (1) nach Anspruch **11** oder 12, **dadurch gekennzeichnet, dass** die Kraftmesseinrichtungen (80) und/oder die Kamera mit der Steuereinheit (70) wirkverbunden sind.

14. Pflegebett (2), umfassend ein Bettgestell nach einem der Ansprüche 1 bis 13 und eine Matratze (3), die auf der Schlafoberflächenbasis (100) angeordnet ist.

15. Verfahren zum Betreiben eines Pflegebetts (2), umfassend ein Bettgestell (1) nach Anspruch 13, **dadurch gekennzeichnet, dass** die Vertikalpositionen der Verstelleinrichtungen (30) und/oder die von den Kraftmesseinrichtungen (80) gemessenen Kräfte von der Steuereinheit (70) erfasst werden und daraus resultierend die Vertikalpositionen durch Ansteuerung der Verstelleinrichtungen (30) angepasst werden.

16. Verfahren zur Kalibrierung eines Pflegebetts (2), umfassend ein Bettgestell (1) nach Anspruch 13, **dadurch gekennzeichnet, dass** durch die Steuereinheit (70) die gemessenen Kräfte der Kraftmesseinrichtungen (80) erfasst werden und das mittels der Steuereinheit (70) eine von der Kamera erfasste Aufnahme ausgewertet wird und daraus die Liegeposition einer im Pflegebett (2) liegenden Person ermittelt wird, so dass die Steuereinheit (70) unter Nutzung eines neuronalen Netzwerks die gemessenen Kräfte und die mittels der Kamera ermittelte Liegeposition korreliert, so dass im Anschluss aus den gemessenen Kräften die Liegeposition wirksam bestimmt werden kann.
